(19) Europäisches Patentamt
European Patent Office
Office européen des brevets

(11) Veröffentlichungsnummer: **0 229 283**
**A1**

(12) # EUROPÄISCHE PATENTANMELDUNG

(21) Anmeldenummer: **86116659.3**

(22) Anmeldetag: **01.12.86**

(51) Int. Cl.³: **C 07 D 239/38**
C 07 D 401/12, C 07 D 403/12
A 01 N 43/54

(30) Priorität: **13.12.85 DE 3544209**

(43) Veröffentlichungstag der Anmeldung:
**22.07.87 Patentblatt 87/30**

(84) Benannte Vertragsstaaten:
**AT BE CH DE FR GB IT LI NL**

(71) Anmelder: **BAYER AG**
**Konzernverwaltung RP Patentabteilung**
**D-5090 Leverkusen 1 Bayerwerk(DE)**

(72) Erfinder: **Sasse, Klaus, Dr.**
**Pützweg 13**
**D-5060 Bergisch Gladbach 2(DE)**

(72) Erfinder: **Fischer, Reiner, Dr.**
**Rembrandtstrasse 15**
**D-4019 Monheim(DE)**

(72) Erfinder: **Hagemann, Hermann, Dr.**
**Kandinsky-Strasse 52**
**D-5090 Leverkusen 1(DE)**

(72) Erfinder: **Santel, Hans-Joachim, Dr.**
**Grünstrasse 9a**
**D-5090 Leverkusen 1(DE)**

(72) Erfinder: **Schmidt, Robert R., Dr.**
**Im Waldwinkel 110**
**D-5060 Bergisch-Gladbach 2(DE)**

(72) Erfinder: **Strang, Harry, Dr.**
**Unterdorfstrasse 6A**
**D-4000 Düsseldorf 31(DE)**

(54) **Pyrimidyl-thio-carbonsäureanilide.**

(57) Die Erfindung betrifft neue Pyrimidyl-thio-carbonsäureanilide Formel (I),

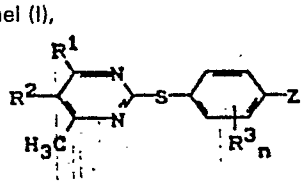

(I)

in welcher

$R^1$ für Wasserstoff, Halogen, für Alkyl, Alkoxy oder Alkylthio mit jeweils 1 bis 6 Kohlenstoffatomen, Trifluormethyl, oder für gegebenenfalls durch Halogen, Methyl, Ethyl, Methoxy oder Trifluormethyl substituiertes Phenyl steht,

$R^2$ für Wasserstoff, Halogen, Alkyl mit 1 bis 6 Kohlenstoffatomen oder Trifluormethyl steht,

$R^3$ für Halogen, Methyl oder Methoxy steht,

n für eine ganze Zahl 0, 1 oder 2, steht, und

Z für den Rest

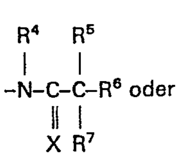 oder 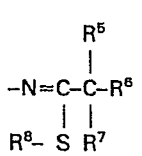

steht,

wobei X,$R^4$,$R^5$,$R^6$,$R^7$ und $R^8$ die in der Beschreibung angegebene Bedeutung haben,

mehrere Verfahren zu ihrer Herstellung und ihre Verwendung als Herbizide.

EP 0 229 283 A1

BAYER AKTIENGESELLSCHAFT 5090 Leverkusen, Bayerwerk
Konzernverwaltung RP
Patentabteilung KM/Ke-c

# Pyrimidyl-thio-carbonsäureanilide

Die vorliegende Erfindung betrifft neue Pyrimidyl-thio-carbonsäureanilide, mehrere Verfahren zu ihrer Herstellung und ihre Verwendung als Herbizide.

Es ist bereits bekannt, daß bestimmte Carbonsäureanilide herbizide Eigenschaften besitzen (vgl. R. Wegler "Chemie der Pflanzenschutz- und Schädlingsbekämpfungsmittel" Bd. 2, Seiten 311-314, Springer-Verlag, Berlin 1970). So kann zum Beispiel das Propionsäure-3,4-dichloranilid zur Unkrautbekämpfung eingesetzt werden. Die Wirkung dieser Verbindung ist gut, jedoch werden bei geringen Aufwandmengen einige Unkräuter nicht immer voll erfaßt. Außerdem läßt auch die Selektivität in manchen Fällen zu wünschen übrig.

Ferner ist bekannt, daß zahlreiche Pyrimidinyl-2-ether und -thioether als Herbizide geeignet sind (vgl. JP-OS 9 474/1967, US-PS 3 126 271 und US-PS 3 250 775). Zum Beispiel können das 2-Phenoxy-4,6-dimethyl-pyrimidin

und das 2- (4-Chlorbenzylmercapto)-4,6-dimethyl-pyrimidin zur Bekämpfung von Unkräutern verwendet werden. Die herbizide Potenz dieser Stoffe ist aber nicht immer ausreichend.

Weiterhin ist bekannt, daß niedere Acylderivate von 4-Pyridyloxy- (bzw. thio)-anilinen herbizide Eigenschaften aufweisen (vgl. DE-OS 2 501 648, JP-OS 55 122 763 und JP-OS 56 123 970). Darüberhinaus sind auch herbizid wirksame Acylderivate von 4-Pyrimidyloxy-anilinen bekannt, die in der 5-Stellung des Pyrimidylrestes durch Halogen oder Trifluormethyl substiutiert sind, dagegen in den Positionen 4 und 6 keinen Substituenten enthalten (vgl. JP-OS 56 029 576). Auch die Wirksamkeit dieser Stoffe ist jedoch für praktische Zwecke nicht immer befriedigend.

Es wurden nun neue Pyrimidyl-thio-carbonsäureanilide der Formel (I),

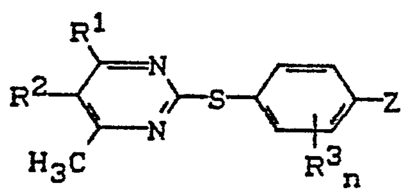

(I)

in welcher

$R^1$ für Wasserstoff, Halogen, für Alkyl, Alkoxy oder Alkylthio mit jeweils 1 bis 6 Kohlenstoffatomen, Trifluormethyl, oder für gegebenenfalls einfach oder mehrfach, gleich oder verschieden durch Halogen, Methyl, Ethyl, Methoxy oder Trifluormethyl substituiertes Phenyl steht,

$R^2$ für Wasserstoff, Halogen, Alkyl mit 1 bis 6 Kohlenstoffatomen oder Trifluormethyl steht,

$R^3$ für Halogen, Methyl oder Methoxy steht,

n für eine ganze Zahl 0, 1 oder 2 steht, und

Z für den Rest

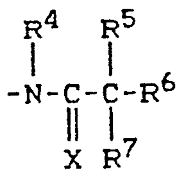

oder

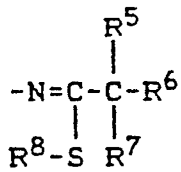

steht,

wobei

X für Sauerstoff oder Schwefel steht,

$R^4$ für Wasserstoff, Hydroxyl, für Alkyl oder Alkoxy mit jeweils 1 bis 6 Kohlenstoffatomen, für Alkenyl oder Alkinyl mit jeweils 3 bis 6 Kohlenstoffatomen steht, mit der Maßgabe, daß $R^4$ nicht für Wasserstoff steht, wenn X für Sauerstoff steht,

$R^5$ für Wasserstoff, Halogen oder für gegebenenfalls einfach oder mehrfach durch Halogen substituiertes Alkyl mit 1 bis 6 Kohlenstoffatomen steht oder

$R^4$ und $R^5$ gemeinsam mit dem Kohlenstoff und dem Stickstoff, an die sie gebunden sind, einen 3- bis 8-gliedrigen heterocyclischen Ring bilden, der gegebenenfalls einfach oder mehrfach, gleich oder verschieden durch Halogen, Methyl oder Ethyl substituiert ist und welcher eine oder mehrere Doppelbindungen enthalten kann,

$R^6$ und $R^7$ unabhängig voneinander für Halogen oder für gegebenenfalls einfach oder mehrfach durch Halogen substituiertes Alkyl mit 1 bis 6 Kohlenstoffatomen stehen oder

$R^6$ und $R^7$ gemeinsam mit dem Kohlenstoffatom, an das sie gebunden sind, einen 3- bis 8-gliedrigen Ring bilden, der außer Kohlenstoff auch Sauerstoff- und/oder Schwefelatome als Ringglieder enthalten kann und der gegebenenfalls einfach oder mehrfach, gleich oder verschieden durch Halogen, Methyl oder Ethyl substituiert ist und welcher eine oder mehrere Doppelbindungen enthalten kann, und

$R^8$ für Wasserstoff, für gegebenenfalls einfach oder mehrfach durch Halogen substituiertes Alkyl mit 1 bis 6 Kohlenstoffatomen, für Alkenyl oder Alkinyl mit jeweils 3 bis 6 Kohlenstoffatomen steht,

gefunden.

Weiterhin wurde gefunden, daß man Pyrimidyl-thiocarbonsäureanilide der Formel (I) mit Hilfe der im folgenden beschriebenen Verfahren erhält:

(a) Man erhält Pyrimidyl-thio-carbonsäureanilide der Formel (Ia),

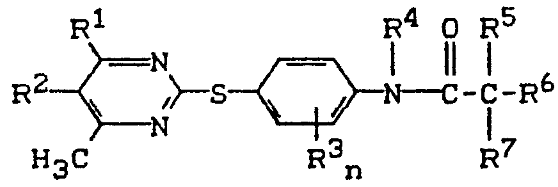

(Ia)

in welcher

$R^1$, $R^2$, $R^3$, $R^4$, $R^5$, $R^6$, $R^7$ und n die oben angegebene Bedeutung haben,

(a-α) wenn man 4-(Pyrimidyl-thio)-aniline der Formel (II),

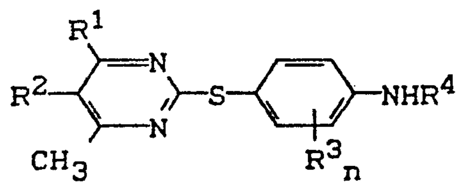

(II)

in welcher

$R^1$, $R^2$, $R^3$, $R^4$ und n die oben angegebene Bedeutung haben,

mit Carbonsäure-Derivaten der Formel (III),

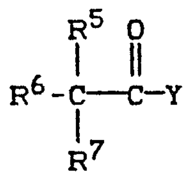

(III)

in welcher

$R^5$, $R^6$ und $R^7$ die oben angegebene Bedeutung haben und

Y für Hydroxyl, Halogen, Acyloxy, Benzolsulfonyl oder Toluolsulfonyl steht,

gegebenenfalls in Gegenwart eines Verdünnungsmittels sowie gegebenenfalls in Gegenwart eines Säurebindemittels umsetzt,

oder

(a-β) die nach Verfahren (a-α) erhältlichen Pyrimidylthio-carbonsäureanilide der Formel (Ia-1),

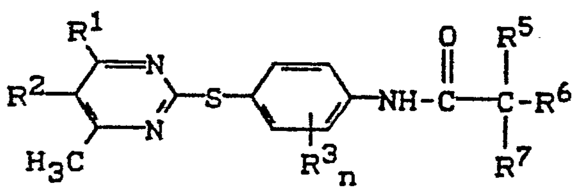

(Ia-1)

in welcher

$R^1$, $R^2$, $R^3$, $R^5$, $R^6$, $R^7$ und n die oben angegebene Bedeutung haben,

mit Alkylierungsmitteln der Formel (IV)

$$R^4 - U \qquad (IV)$$

in welcher

$R^4$ die oben angegebene Bedeutung hat und

U für eine elektronenanziehende Abgangsgruppe steht,

gegebenenfalls in Gegenwart eines Verdünnungsmittels sowie gegebenenfalls in Gegenwart eines Säurebindemittels umsetzt,

oder man erhält

(b) Pyrimidyl-thio-carbonsäureanilide der Formel (Ib),

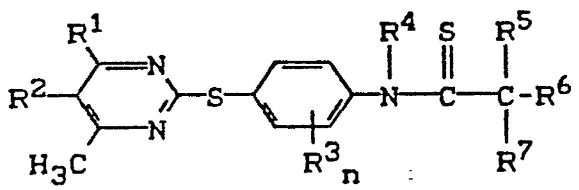

(Ib)

in welcher

$R^1$, $R^2$, $R^3$, $R^4$, $R^5$, $R^6$, $R^7$ und n die oben angegebene Bedeutung haben,

wenn man die nach Verfahren (a/Variante α und β) erhältlichen Pyrimidyl-thio-carbonsäureanilide der Formel (Ia),

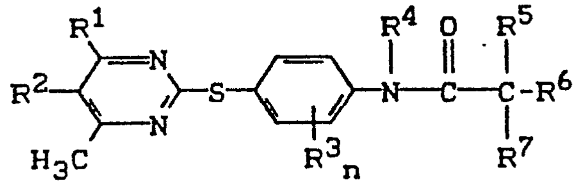

(Ia)

in welcher

$R^1$, $R^2$, $R^3$, $R^4$, $R^5$, $R^6$, $R^7$ und n die oben angegebene Bedeutung haben,

mit Schwefelungsagentien in Gegenwart eines Verdünnungsmittels umsetzt,

oder man erhält

(c) Pyrimidyl-thio-carbonsäureanilide der Formel (Ic),

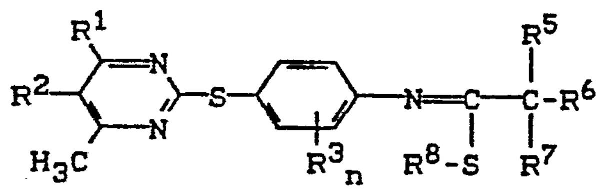

(Ic)

in welcher

$R^1$, $R^2$, $R^3$, $R^5$, $R^6$, $R^7$, $R^8$ und n die oben angegebene Bedeutung haben,

wenn man die nach Verfahren (b) erhältlichen Pyrimidyl-thio-carbonsäureanilide der Formel (Ib), mit Alkylierungsmitteln der Formel (V);

$$R^8 - U \qquad (V)$$

in welcher

$R^8$ und U die oben angegebene Bedeutung haben,

gegebenenfalls in Gegenwart eines Verdünnungsmittels und gegebenenfalls in Gegenwart eines Säurebindemittels umsetzt.

Schließlich wurde gefunden, daß die neuen Pyrimidyl-thio-carbonsäureanilide der Formel (I) herbizide, insbesondere auch selektiv-herbizide Eigenschaften besitzen.

Überraschenderweise zeigen die erfindungsgemäßen Pyrimidyl-thio-carbonsäureanilide der Formel (I) wesentlich bessere herbizide Eigenschaften als konstitutionell ähnliche vorbekannte Stoffe gleicher Wirkungsart.

Die erfindungsgemäßen Pyrimidyl-thio-carbonsäureanilide sind durch die Formel (I) definiert. Bevorzugt sind Verbindungen der Formel (I), bei welchen

$R^1$ für Wasserstoff, Fluor, Chlor, Brom, für jeweils geradkettiges oder verzweigtes Alkyl, Alkoxy oder Alkylthio mit jeweils 1 bis 4 Kohlenstoffatomen oder für Trifluormethyl steht,

$R^2$ für Wasserstoff, Fluor, Chlor, Brom, geradkettiges oder verzweigtes Alkyl mit 1 bis 4 Kohlenstoffatomen oder Trifluormethyl steht,

$R^3$ für Fluor, Chlor, Brom, Methyl oder Methoxy steht,

n für eine ganze Zahl 0, 1 oder 2 steht und

Z für den Rest

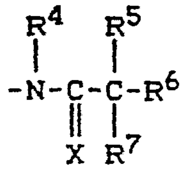 oder 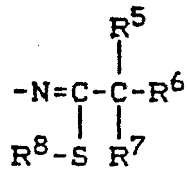

steht,

wobei

X für Sauerstoff oder Schwefel steht,

$R^4$ für Wasserstoff, Hydroxyl, für jeweils geradkettiges oder verzweigtes Alkyl oder Alkoxy mit jeweils 1 bis 4 Kohlenstoffatomen, für jeweils geradkettiges oder verzweigtes Alkenyl oder Alkinyl mit jeweils 3 bis 4 Kohlenstoffatomen steht,

mit der Maßgabe, daß $R^4$ nicht für Wasserstoff steht, wenn X für Sauerstoff steht,

$R^5$ für Wasserstoff, Fluor, Chlor, Brom oder für geradkettiges oder verzweigtes gegebenenfalls einfach bis neunfach, gleich oder verschieden durch Fluor, Chlor und Brom substituiertes Alkyl mit 1 bis 4 Kohlenstoffatomen steht oder

$R^4$ und $R^5$ gemeinsam mit dem Kohlenstoff bzw. dem Stickstoff, an die sie gebunden sind, einen 3- bis 8-gliedrigen heterocyclischen Ring bilden, der gegebenenfalls einfach bis zwölffach, gleich oder verschieden durch Methyl und Ethyl substituiert ist und welcher eine, zwei oder drei oder vier Doppelbindungen enthalten kann,

$R^6$ und $R^7$ unabhängig voneinander für Fluor, Chlor, Brom oder für geradkettiges oder verzweigtes gegebenenfalls einfach bis neunfach, gleich oder verschieden durch Fluor und Chlor substituiertes Alkyl mit 1 bis 4 Kohlenstoffatomen stehen oder

$R^6$ und $R^7$ gemeinsam mit dem Kohlenstoffatom, an das sie gebunden sind, einen 3- bis 8-gliedrigen Ring bilden, der außer Kohlenstoff gegebenenfalls 1 oder 2 Sauerstoff- und/oder Schwefelatome als Ringglieder enthält und der gegebenenfalls einfach bis zwölffach, gleich oder verschieden durch Fluor, Chlor, Methyl und Ethyl substituiert ist und welcher eine oder zwei Doppelbindungen enthalten kann, und

$R^8$ für Wasserstoff, für gegebenenfalls einfach bis fünffach, gleich oder verschieden durch Fluor oder Chlor substituiertes geradkettiges oder verzweigtes Alkyl mit 1 bis 4 Kohlenstoffatomen, oder für jeweils geradkettiges oder verzweigtes Alkenyl oder Alkinyl mit jeweils 3 bis 6 Kohlenstoffatomen steht.

Besonders bevorzugt sind Verbindungen der Formel (I), bei welchen

$R^1$ für Wasserstoff, Fluor, Chlor, Brom, Methyl, Ethyl, Methoxy, Ethoxy, Methylthio, Ethylthio oder Trifluormethyl steht,

$R^2$ für Wasserstoff, Fluor, Chlor, Brom, Methyl, Ethyl oder Trifluormethyl steht,

$R^3$ für Fluor, Chlor, Brom, Methyl oder Methoxy steht,

n für eine ganze Zahl 0, 1 oder 2 steht,

Z für den Rest

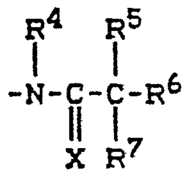 oder 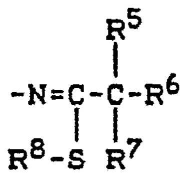

steht,

wobei

X für Sauerstoff oder Schwefel steht,

$R^4$ für Wasserstoff, Hydroxyl, Methyl, Ethyl, Methoxy, Ethoxy, Allyl oder Propargyl steht,

mit der Maßgabe, daß $R^4$ nicht für Wasserstoff steht, wenn X für Sauerstoff steht,

$R^5$ für Wasserstoff, Fluor, Chlor, Brom, Methyl oder Ethyl steht, oder

$R^4$ und $R^5$ gemeinsam mit dem Kohlenstoff bzw. dem Stickstoff, an die sie gebunden sind, einen 3- bis 8-gliedrigen heterocyclischen Ring bilden, der gegebenenfalls einfach bis sechsfach, gleich oder verschieden durch Methyl oder Ethyl substituiert ist,

$R^6$ und $R^7$ unabhängig voneinander für Fluor, Chlor, Brom, Methyl, Ethyl oder iso-Propyl stehen, oder

$R^6$ und $R^7$ gemeinsam mit dem Kohlenstoffatom, an das sie gebunden sind, einen 3- bis 8-gliedrigen Ring bilden, der außer Kohlenstoff gegebenenfalls 1 oder 2 Sauerstoff- und/oder Schwefelatome als Ringglieder enthält und der gegebenenfalls einfach bis dreifach, gleich oder verschieden durch Fluor, Chlor, Methyl oder Ethyl substituiert ist, und

$R^8$ für Wasserstoff, für gegebenenfalls einfach bis fünffach, gleich oder verschieden durch Fluor oder Chlor substituiertes Methyl, Ethyl oder iso-Propyl, für Allyl oder Propargyl steht.

Im einzelnen seien außer den bei den Herstellungsbeispielen genannten Verbindungen die folgenden Pyrimidyl-thiocarbonsäureanilide der Formel (I) genannt:

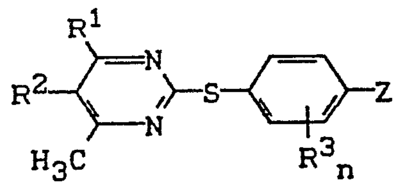

(I)

Tabelle 1

Z = 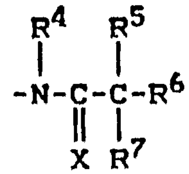

| $R^1$ | $R^2$ | $R^3$ | $R^4$ | $R^5$ | $R^6$ | $R^7$ | X |
|---|---|---|---|---|---|---|---|
| $CH_3$ | H | H | $C_2H_5$ | $CH_3$ | $CH_3$ | $CH_3$ | O |
| $CH_3$ | H | H | $i\text{-}C_3H_7$ | $CH_3$ | $CH_3$ | $CH_3$ | O |
| $CH_3$ | H | H | $CH_2\text{-}CH{=}CH_2$ | $CH_3$ | $CH_3$ | $CH_3$ | O |
| $CH_3$ | H | H | $CH_2\text{-}C{\equiv}CH$ | $CH_3$ | $CH_3$ | $CH_3$ | O |
| $CH_3$ | H | H | $-(CH_2)_4-$ | | $CH_3$ | $CH_3$ | O |
| $CH_3$ | H | H | $-(CH_2)_3-$ | | $CH_3$ | $CH_3$ | O |
| $CH_3$ | H | H | $CH_3$ | $CH_3$ | $CH_3$ | H | O |
| $CH_3$ | H | H | $CH_3$ | $C_2H_5$ | $C_2H_5$ | H | O |
| $CH_3$ | H | Cl | $CH_3$ | $CH_3$ | $CH_3$ | $CH_3$ | O |
| $CH_3$ | H | Cl | $-CH_2\text{-}CH{=}CH_2$ | $CH_3$ | $CH_3$ | $CH_3$ | O |
| $CH_3$ | H | Cl | $-(CH_2)_3-$ | | H | H | O |
| $CH_3$ | H | Cl | $-(CH_2)_3-$ | | $CH_3$ | $CH_3$ | O |
| $CH_3$ | H | Cl | $-(CH_2)_4-$ | | H | H | O |
| $CH_3$ | H | Cl | $-(CH_2)_4-$ | | $CH_3$ | $CH_3$ | O |
| $CH_3$ | H | Cl | $CH_3$ | $CH_3$ | $CH_3$ | H | O |
| $CH_3$ | H | Cl | $-CH_2\text{-}CH{=}CH_2$ | $CH_3$ | $CH_3$ | H | O |

Tabelle 1 (Fortsetzung)

| $R^1$ | $R^2$ | $R^3$ | $R^4$ | $R^5$ | $R^6$ | $R^7$ | X |
|---|---|---|---|---|---|---|---|
| $CH_3$ | H | Cl | $CH_3$ | $C_2H_5$ | $C_2H_5$ | H | O |
| H | $C_2H_5$ | Cl | $CH_3$ | $CH_3$ | $CH_3$ | $CH_3$ | O |
| H | $C_2H_5$ | $CH_3$ | $-(CH_2)_3-$ | | $CH_3$ | $CH_3$ | O |
| H | $C_2H_5$ | $OCH_3$ | $-CH_2-CH=CH_2$ | $CH_3$ | $CH_3$ | $CH_3$ | O |
| H | H | H | $CH_3$ | $CH_3$ | $CH_3$ | $CH_3$ | O |
| H | H | H | $-(CH_2)_3-$ | | $CH_3$ | $CH_3$ | O |
| H | H | H | $-CH_2-CH=CH_2$ | $C_2H_5$ | $C_2H_5$ | H | O |
| H | H | Cl | $CH_3$ | $CH_3$ | $CH_3$ | $CH_3$ | O |
| H | H | $CH_3$ | $-(CH_2)_4-$ | | $CH_3$ | $CH_3$ | O |
| H | H | $OCH_3$ | $-CH_2-CH=CH_2$ | $CH_3$ | $CH_3$ | H | O |
| $CH_3$ | Cl | H | $CH_3$ | $CH_3$ | $CH_3$ | $CH_3$ | O |
| $CH_3$ | Cl | H | $-(CH_2)_3$ | | $CH_3$ | $CH_3$ | O |
| $CH_3$ | Cl | Cl | $CH_3$ | $CH_3$ | $CH_3$ | $CH_3$ | O |
| $CH_3$ | H | H | H | $CH_3$ | $CH_3$ | $i-C_3H_7$ | S |
| $CH_3$ | H | H | H | $CH_3$ | $-(CH_2)_4-$ | | S |
| $CH_3$ | H | H | $-(CH_2)_4$ | | $CH_3$ | $CH_3$ | S |
| $CH_3$ | H | Cl | H | $CH_3$ | $CH_3$ | $CH_3$ | S |
| $CH_3$ | H | H | H | $CH_3$ | $-(CH_2)_5-$ | H | S |

<u>Tabelle 1</u> (Fortsetzung)

| $R^1$ | $R^2$ | $R^3$ | $R^4$ | $R^5$ | $R^6$ | $R^7$ | X |
|---|---|---|---|---|---|---|---|
| $CH_3$ | H | $OCH_3$ | $CH_3$ | $C_2H_5$ | $C_2H_5$ | H | O |
| $CH_3$ | H | $OCH_3$ | $-CH_2-CH=CH_2$ | $C_2H_5$ | $C_2H_5$ | H | O |
| $CH_3$ | $CH_3$ | H | $CH_3$ | $CH_3$ | $CH_3$ | $CH_3$ | O |
| $CH_3$ | $CH_3$ | H | $-CH_2-CH=CH_2$ | $CH_3$ | $CH_3$ | $CH_3$ | O |
| $CH_3$ | $CH_3$ | H | $-(CH_2)_3-$ | | $CH_3$ | $CH_3$ | O |
| $CH_3$ | $CH_3$ | Cl | $CH_3$ | $CH_3$ | $CH_3$ | $CH_3$ | O |
| $CH_3$ | $CH_3$ | $CH_3$ | $-(CH_2)_3-$ | | $CH_3$ | $CH_3$ | O |
| $CH_3$ | $CH_3$ | H | $-CH_2-CH=CH_2$ | $CH_3$ | $CH_3$ | $CH_3$ | O |
| $CH_3$ | H | $CH_3$ | $CH_3$ | $CH_3$ | $CH_3$ | $CH_3$ | O |
| $CH_3$ | H | $CH_3$ | $-CH_2-CH=CH_2$ | $CH_3$ | $CH_3$ | $CH_3$ | O |
| $CH_3$ | H | $CH_3$ | $-(CH_2)_3-$ | | $CH_3$ | $CH_3$ | O |
| $CH_3$ | H | $CH_3$ | $-(CH_2)_4-$ | | $CH_3$ | $CH_3$ | O |
| $CH_3$ | H | $CH_3$ | $-CH_3$ | $CH_3$ | $CH_3$ | H | O |
| $CH_3$ | H | $CH_3$ | $-CH_2-CH=CH_2$ | $CH_3$ | $CH_3$ | H | O |
| $CH_3$ | H | $CH_3$ | $-CH_3$ | $C_2H_5$ | $C_2H_5$ | H | O |
| $CH_3$ | H | $CH_3$ | $-CH_2-CH=CH_2$ | $C_2H_5$ | $C_2H_5$ | H | O |
| $CH_3$ | H | $OCH_3$ | $CH_3$ | $CH_3$ | $CH_3$ | $CH_3$ | O |
| $CH_3$ | H | $OCH_3$ | $-CH_2CH=CH_2$ | $CH_3$ | $CH_3$ | $CH_3$ | O |

Tabelle 1 (Fortsetzung)

| $R^1$ | $R^2$ | $R^3$ | $R^4$ | $R^5$ | $R^6$ | $R^7$ | X |
|---|---|---|---|---|---|---|---|
| $CH_3$ | H | $OCH_3$ | $-(CH_2)_3-$ | | $CH_3$ | $CH_3$ | O |
| $CH_3$ | H | $OCH_3$ | $-(CH_2)_4-$ | | $CH_3$ | $CH_3$ | O |
| $CH_3$ | H | $OCH_3$ | $CH_3$ | $CH_3$ | $CH_3$ | H | O |
| $CH_3$ | H | $OCH_3$ | $-CH_2-CH=CH_2$ | $CH_3$ | $CH_3$ | H | O |
| $CH_3$ | H | $OCH_3$ | $CH_3$ | $C_2H_5$ | $C_2H_5$ | H | O |
| $CH_3$ | H | $OCH_3$ | $-CH_2-CH=CH_2$ | $C_2H_5$ | $C_2H_5$ | H | O |
| $CH_3$ | $CH_3$ | H | $CH_3$ | $CH_3$ | $CH_3$ | $CH_3$ | O |
| $CH_3$ | $CH_3$ | H | $-CH_2-CH=CH_2$ | $CH_3$ | $CH_3$ | $CH_3$ | O |
| $CH_3$ | $CH_3$ | H | $-(CH_2)_3-$ | | $CH_3$ | $CH_3$ | O |
| $CH_3$ | $CH_3$ | Cl | $CH_3$ | $CH_3$ | $CH_3$ | $CH_3$ | O |
| $CH_3$ | $CH_3$ | $CH_3$ | $-(CH_2)_3-$ | | $CH_3$ | $CH_3$ | O |
| $CH_3$ | $CH_3$ | $OCH_3$ | $-CH_2-CH=CH_2$ | $CH_3$ | $CH_3$ | $CH_3$ | O |
| H | $C_2H_5$ | H | $CH_3$ | $CH_3$ | $CH_3$ | $CH_3$ | O |
| H | $C_2H_5$ | H | $-(CH_2)_3-$ | | $CH_3$ | $CH_3$ | O |
| H | $C_2H_5$ | H | $-CH_2-CH=CH_2$ | $CH_3$ | $CH_3$ | H | O |

Tabelle 1 (Fortsetzung)

| $R^1$ | $R^2$ | $R^3$ | $R^4$ | $R^5$ | $R^6$ | $R^7$ | X |
|---|---|---|---|---|---|---|---|
| $CH_3$ | H | Cl | $CH_3$ | $CH_3$ | $CH_3$ | H | O |
| $CH_3$ | H | Cl | $-CH_2-CH=CH_2$ | $CH_3$ | $CH_3$ | H | O |
| $CH_3$ | H | Cl | $CH_3$ | $C_2H_5$ | $C_2H_5$ | H | O |
| $CH_3$ | H | $CH_3$ | $CH_3$ | $CH_3$ | $CH_3$ | $CH_3$ | O |
| $CH_3$ | H | $CH_3$ | $-CH_2-CH=CH_2$ | $CH_3$ | $CH_3$ | $CH_3$ | O |
| $CH_3$ | H | $CH_3$ | $-(CH_2)_3-$ | | $CH_3$ | $CH_3$ | O |
| $CH_3$ | H | $CH_3$ | $-(CH_2)_4-$ | | $CH_3$ | $CH_3$ | O |
| $CH_3$ | H | $CH_3$ | $CH_3$ | $CH_3$ | $CH_3$ | H | O |
| $CH_3$ | H | $CH_3$ | $-CH_2-CH=CH_2$ | $CH_3$ | $CH_3$ | H | O |
| $CH_3$ | H | $CH_3$ | $-CH_3$ | $C_2H_5$ | $C_2H_5$ | H | O |
| $CH_3$ | H | $CH_3$ | $-CH_2-CH=CH_2$ | $C_2H_5$ | $C_2H_5$ | H | O |
| $CH_3$ | H | $OCH_3$ | $CH_3$ | $CH_3$ | $CH_3$ | $CH_3$ | O |
| $CH_3$ | H | $OCH_3$ | $-CH_2CH=CH_2$ | $CH_3$ | $CH_3$ | $CH_3$ | O |
| $CH_3$ | H | $OCH_3$ | $-(CH_2)_3-$ | | $CH_3$ | $CH_3$ | O |
| $CH_3$ | H | $OCH_3$ | $-(CH_2)_4-$ | | $CH_3$ | $CH_3$ | O |
| $CH_3$ | H | $OCH_3$ | $CH_3$ | $CH_3$ | $CH_3$ | H | O |
| $CH_3$ | H | $OCH_3$ | $-CH_2-CH=CH_2$ | $CH_3$ | $CH_3$ | H | O |

Tabelle 1 (Fortsetzung)

| $R^1$ | $R^2$ | $R^3$ | $R^4$ | $R^5$ | $R^6$ | $R^7$ | X |
|---|---|---|---|---|---|---|---|
| $CH_3$ | H | Cl | H | $CH_3$ | $CH_3$ | $i-C_3H_7$ | S |
| $CH_3$ | H | Cl | H | $CH_3$ | $CH_3$ | $-CH=CH_2$ | S |
| $CH_3$ | H | Cl | H | $CH_3$ | $-(CH_2)_4-$ | | S |
| $CH_3$ | H | Cl | $-(CH_2)_3-$ | | $CH_3$ | $CH_3$ | S |
| $CH_3$ | H | $CH_3$ | H | $CH_3$ | $CH_3$ | $CH_3$ | S |
| $CH_3$ | H | $CH_3$ | H | $CH_3$ | $CH_3$ | $i-C_3H_7$ | S |
| $CH_3$ | H | $CH_3$ | H | $CH_3$ | $CH_3$ | $-CH=CH_2$ | S |
| $CH_3$ | H | $CH_3$ | H | $CH_3$ | $-CH_2)_4-$ | | S |
| $CH_3$ | H | $CH_3$ | $-(CH_2)_3$ | | $CH_3$ | $CH_3$ | S |
| $CH_3$ | H | $OCH_3$ | H | $CH_3$ | $CH_3$ | $CH_3$ | S |
| $CH_3$ | H | $OCH_3$ | H | $CH_3$ | $CH_3$ | $i-C_3H_7$ | S |
| $CH_3$ | H | $OCH_3$ | H | $CH_3$ | $CH_3$ | $-CH=CH_2$ | S |
| $CH_3$ | H | $OCH_3$ | H | $CH_3$ | $-(CH_2)_4-$ | | S |
| $CH_3$ | H | $OCH_3$ | $-(CH_2)_3$ | | $CH_3$ | $CH_3$ | S |
| $CH_3$ | $CH_3$ | H | H | $CH_3$ | $CH_3$ | $CH_3$ | S |

Tabelle 1 (Fortsetzung)

| $R^1$ | $R^2$ | $R^3$ | $R^4$ | $R^5$ | $R^6$ | $R^7$ | X |
|---|---|---|---|---|---|---|---|
| $CH_3$ | $CH_3$ | H | H | $CH_3$ | $CH_3$ | $i-C_3H_7$ | S |
| $CH_3$ | $CH_3$ | H | H | $CH_3$ | $CH_3$ | $-CH=CH_2$ | S |
| $CH_3$ | $CH_3$ | H | H | $CH_3$ | $-(CH_2)_4-$ | | S |
| $CH_3$ | $CH_3$ | H | $-(CH_2)_3-$ | | $CH_3$ | $CH_3$ | S |
| H | $C_2H_5$ | H | H | $CH_3$ | $CH_3$ | $CH_3$ | S |
| H | $C_2H_5$ | H | H | $CH_3$ | $CH_3$ | $i-C_3H_7$ | S |
| H | $C_2H_5$ | H | H | $CH_3$ | $CH_3$ | $-CH=CH_2$ | S |
| H | $C_2H_5$ | H | H | $CH_3$ | $-(CH_2)_4-$ | | S |
| H | $C_2H_5$ | H | $-(CH_2)_3-$ | | $CH_3$ | $CH_3$ | S |
| H | H | H | H | $CH_3$ | $CH_3$ | $CH_3$ | S |
| H | H | H | H | $CH_3$ | $CH_3$ | $i-C_3H_7$ | S |
| H | H | H | H | $CH_3$ | $CH_3$ | $-CH=CH_2$ | S |
| H | H | H | H | $CH_3$ | $-(CH_2)_4$ | | S |
| H | H | H | $-(CH_2)_3-$ | | $CH_3$ | $CH_3$ | S |
| $CH_3$ | Cl | | H | $CH_3$ | $CH_3$ | $CH_3$ | S |
| $CH_3$ | Cl | | H | $CH_3$ | $CH_3$ | $CH_3$ | S |
| $CH_3$ | Cl | | H | $CH_3$ | $CH_3$ | $CH_3$ | S |
| $CH_3$ | Cl | H | H | $CH_3$ | $-(CH_2)_4-$ | | S |
| $CH_3$ | Cl | | $-(CH_2)_3$ | | $CH_3$ | $CH_3$ | S |

Tabelle 2

Z = 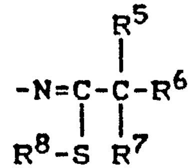

| $R^1$ | $R^2$ | $R^3$ | $R^5$ | $R^6$ | $R^7$ | $R^8$ |
|---|---|---|---|---|---|---|
| $CH_3$ | H | H | $CH_3$ | $CH_3$ | $CH_3$ | $CH_3$ |
| $CH_3$ | H | H | $CH_3$ | $CH_3$ | $CH_3$ | $C_2H_5$ |
| $CH_3$ | H | H | $CH_3$ | $CH_3$ | $CH_3$ | $i-C_3H_7$ |
| $CH_3$ | H | H | $CH_3$ | $CH_3$ | $CH_3$ | $CH_2-CH=CH_2$ |
| $CH_3$ | H | H | $CH_3$ | $CH_3$ | $CH_3$ | $CH_2-C≡CH$ |
| $CH_3$ | H | H | $CH_3$ | $CH_3$ | $i-C_3H_7$ | $CH_3$ |
| $CH_3$ | H | H | $CH_3$ | $CH_3$ | $-CH=CH_2$ | $CH_3$ |
| $CH_3$ | H | H | $CH_3$ | $CH_3$ | $i-C_3H_7$ | $i-C_3H_7$ |
| $CH_3$ | H | H | $CH_3$ | $CH_3$ | $-CH=CH_2$ | $i-C_3H_7$ |
| $CH_3$ | H | H | $CH_3$ | $CH_3$ | $i-C_3H_7$ | $CH_2-CH=CH_2$ |
| $CH_3$ | H | H | $CH_3$ | $CH_3$ | $-CH=CH_2$ | $CH_2-CH=CH_2$ |
| $CH_3$ | H | H | $CH_3$ | $CH_3$ | $i-C_3H_7$ | $CH_2-C≡CH$ |
| $CH_3$ | H | H | $CH_3$ | $CH_3$ | $-CH=CH_2$ | $CH_2-C≡CH$ |
| $CH_3$ | H | Cl | $CH_3$ | $CH_3$ | $CH_3$ | $CH_3$ |

Tabelle 2 (Fortsetzung)

| $R^1$ | $R^2$ | $R^3$ | $R^5$ | $R^6$ | $R^7$ | $R^8$ |
|---|---|---|---|---|---|---|
| $CH_3$ | H | Cl | $CH_3$ | $CH_3$ | $CH_3$ | $i\text{-}C_3H_7$ |
| $CH_3$ | H | Cl | $CH_3$ | $CH_3$ | $CH_3$ | $CH_2\text{-}CH{=}CH_2$ |
| $CH_3$ | H | $CH_3$ | $CH_3$ | $CH_3$ | $CH_3$ | $CH_3$ |
| $CH_3$ | H | $CH_3$ | $CH_3$ | $CH_3$ | $CH_3$ | $i\text{-}C_3H_7$ |
| $CH_3$ | H | $CH_3$ | $CH_3$ | $CH_3$ | $CH_3$ | $CH_2\text{-}CH{=}CH_2$ |
| $CH_3$ | H | $OCH_3$ | $CH_3$ | $CH_3$ | $CH_3$ | $CH_3$ |
| $CH_3$ | H | $OCH_3$ | $CH_3$ | $CH_3$ | $CH_3$ | $i\text{-}C_3H_7$ |
| $CH_3$ | H | $OCH_3$ | $CH_3$ | $CH_3$ | $CH_3$ | $CH_2\text{-}CH{=}CH_2$ |
| $CH_3$ | $CH_3$ | H | $CH_3$ | $CH_3$ | $CH_3$ | $CH_3$ |
| $CH_3$ | $CH_3$ | H | $CH_3$ | $CH_3$ | $CH_3$ | $i\text{-}C_3H_5$ |
| $CH_3$ | $CH_3$ | H | $CH_3$ | $CH_3$ | $CH_3$ | $CH_2\text{-}CH{=}CH_2$ |
| $CH_3$ | $CH_3$ | Cl | $CH_3$ | $CH_3$ | $CH_3$ | $CH_3$ |
| $CH_3$ | $CH_3$ | Cl | $CH_3$ | $CH_3$ | $CH_3$ | $i\text{-}C_3H_7$ |
| $CH_3$ | $CH_3$ | Cl | $CH_3$ | $CH_3$ | $CH_3$ | $CH_2CH{=}CH_2$ |
| $CH_3$ | $CH_3$ | $CH_3$ | $CH_3$ | $CH_3$ | $CH_3$ | $CH_3$ |
| $CH_3$ | $CH_3$ | $CH_3$ | $CH_3$ | $CH_3$ | $CH_3$ | $i\text{-}C_3H_7$ |

Tabelle 2 (Fortsetzung)

| $R^1$ | $R^2$ | $R^3$ | $R^5$ | $R^6$ | $R^7$ | $R^8$ |
|---|---|---|---|---|---|---|
| $CH_3$ | $CH_3$ | $CH_3$ | $CH_3$ | $CH_3$ | $CH_3$ | $CH_2-CH=CH_2$ |
| $CH_3$ | $CH_3$ | $OCH_3$ | $CH_3$ | $CH_3$ | $CH_3$ | $CH_3$ |
| $CH_3$ | $CH_3$ | $OCH_3$ | $CH_3$ | $CH_3$ | $CH_3$ | $i-C_3H_7$ |
| $CH_3$ | $CH_3$ | $OCH_3$ | $CH_3$ | $CH_3$ | $CH_3$ | $-CH_2-CH=CH_2$ |
| H | $C_2H_5$ | H | $CH_3$ | $CH_3$ | $CH_3$ | $CH_3$ |
| H | $C_2H_5$ | H | $CH_3$ | $CH_3$ | $CH_3$ | $i-C_3H_7$ |
| H | $C_2H_5$ | H | $CH_3$ | $CH_3$ | $CH_3$ | $-CH_2-CH=CH_2$ |
| H | $C_2H_5$ | Cl | $CH_3$ | $CH_3$ | $CH_3$ | $CH_3$ |
| H | $C_2H_5$ | $CH_3$ | $CH_3$ | $CH_3$ | $CH_3$ | $CH_3$ |
| H | $C_2H_5$ | $OCH_3$ | $CH_3$ | $CH_3$ | $CH_3$ | $CH_3$ |
| H | H | H | $CH_3$ | $CH_3$ | $CH_3$ | $CH_3$ |
| H | H | H | $CH_3$ | $CH_3$ | $CH_3$ | i-$C_3$H7 |
| H | H | H | $CH_3$ | $CH_3$ | $CH_3$ | $-CH_2CH=CH_2$ |
| $CH_3$ | Cl | H | $CH_3$ | $CH_3$ | $CH_3$ | $CH_3$ |
| $CH_3$ | Cl | H | $CH_3$ | $CH_3$ | $CH_3$ | $i-C_3H_7$ |

Verwendet man beispielsweise 4-[(4,6-Dimethyl-2-mercapto)-pyrimidyl-N-methyl-anilin und Pivaloylchlorid als Ausgangsstoffe, so läßt sich der Reaktionsverlauf des erfindungsgemäßen Verfahrens (a-α) durch das folgende Formelschema darstellen:

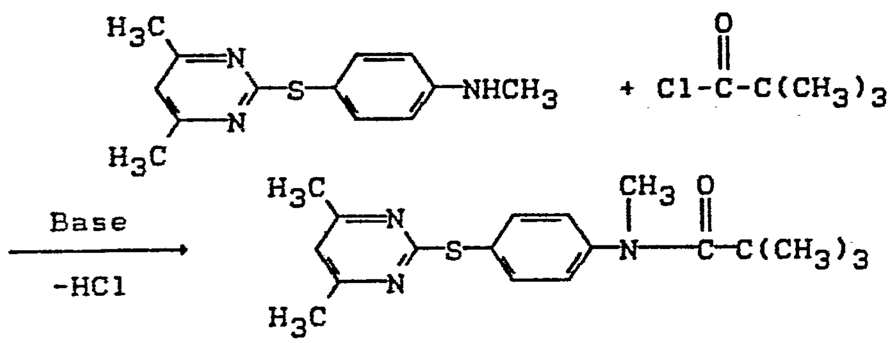


Verwendet man beispielsweise 4-[(4,6-Dimethyl-2-mercapto)-pyrimidyl]-(2-ethylbuttersäure)-anilid und Alkylbromid als Ausgangsstoffe, so läßt sich der Reaktionsverlauf des erfindungsgemäßen Verfahrens (a-β) durch das folgende Formelschema darstellen:

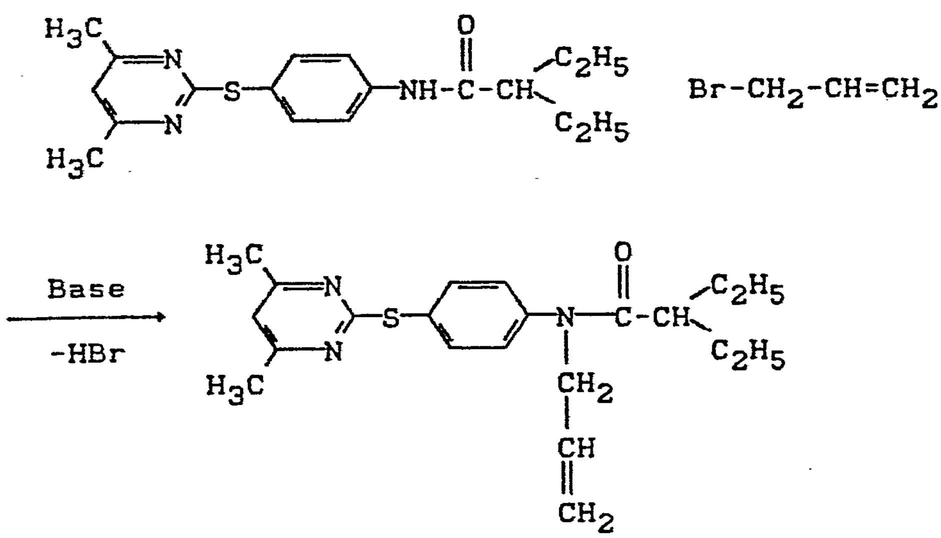

Verwendet man beispielsweise 4-[(4,6-Dimethyl-2-mercapto)-pyrimidyl]-pivaloylanilid und das Lawesson-Reagenz als Ausgangsstoffe, so läßt sich der Reaktionsverlauf des erfindungsgemäßen Verfahrens (b) durch das folgende Formelschema darstellen:

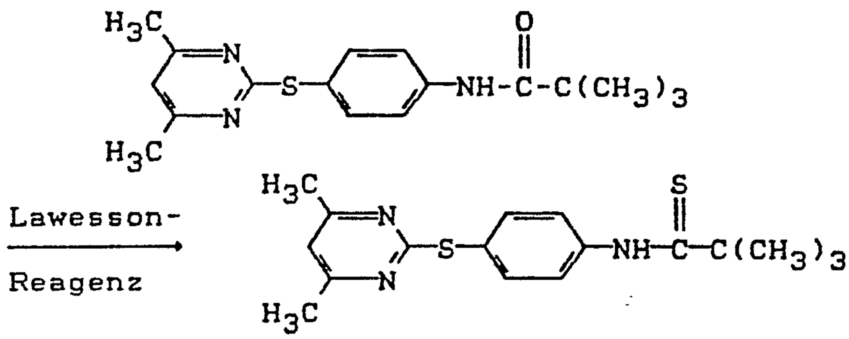


Verwendet man beispielsweise 4-[(4,6-Dimethyl-2-mercapto)-pyrimidyl]-pyvaloylthioanilid und Methyljodid als Ausgangsstoffe, so läßt sich der Reaktionsverlauf des erfindungsgemäßen Verfahrens (c) durch das folgende Formelschema darstellen:

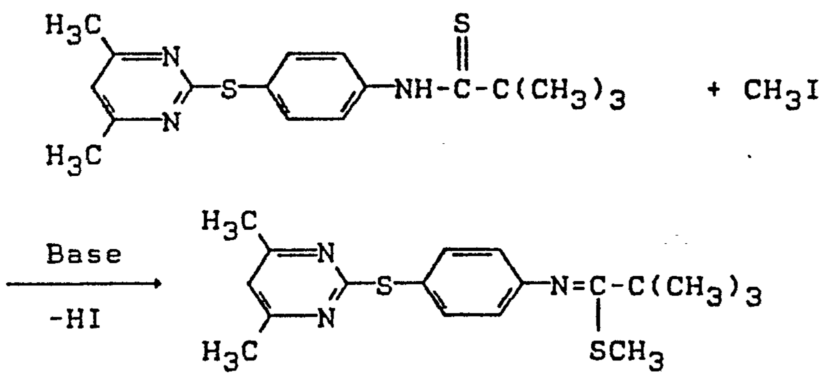

Die bei dem erfindungsgemäßen Verfahren (a-α) als Ausgangsstoffe benötigten 4-(Pyrimidyl-thio)-aniline sind durch die Formel (II) allgemein definiert. In der Formel (II) haben $R^1$, $R^2$, $R^3$, $R^4$ und n vorzugsweise diejenigen Bedeutungen, die bereits im Zusammenhang mit der Beschreibung der erfindungsgemäßen Stoffe der Formel (I) vorzugsweise für diese Reste bzw. diesen Index genannt wurden.

Die 4-(Pyrimidyl-thio)-aniline der Formel (II) sind teilweise bekannt. Sie lassen sich herstellen, indem man

(d) Pyrimidin-Derivate der Formel (VI)

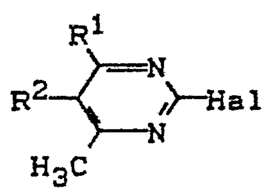

(VI)

in welcher

$R^1$ und $R^2$ die oben angegebene Bedeutung haben, und

Hal für Halogen steht,

mit 4-Amino-thiophenolen der Formel (VII),

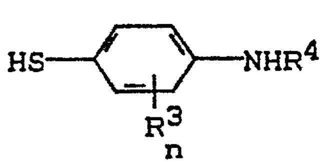

(VII)

in welcher

$R^3$, $R^4$ und n die oben angegebene Bedeutung haben,

gegebenenfalls in Gegenwart eines Säurebindemittels sowie gegebenenfalls in Gegenwart eines Verdünnungsmittels umsetzt,

oder

(e) 2-(4-Nitro-thiophenoxy)-pyrimidin-Derivate der Formel (VIII),

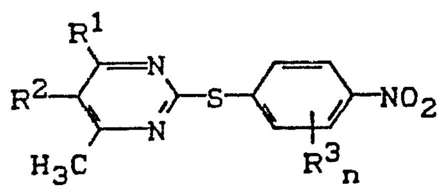

(VIII)

in welcher

$R^1$, $R^2$, $R^3$ und n die oben angegebene Bedeutung haben,

gegebenenfalls in Gegenwart eines Verdünnungsmittels nach üblichen Methoden reduziert.

Die bei dem obigen Verfahren (d) als Ausgangsstoffe benötigten Pyrimidin-Derivate sind durch die Formel (VI) definiert. In dieser Formel haben $R^1$ und $R^2$ vorzugsweise diejenigen Bedeutungen, die bereits im Zusammenhang mit der Beschreibung der erfindungsgemäßen Stoffe der Formel (I) vorzugsweise für diese Reste genannt wurden. Hal steht vorzugsweise für Fluor, Chlor oder Brom.

Die Pyrimidin-Derivate der Formel (VI) sind bekannt oder lassen sich nach im Prinzip bekannten Methoden in einfacher Weise herstellen. So erhält man Pyrimidin-Derivate der Formel (VI) zum Beispiel dadurch, daß man 2-Hydroxypyrimidin-Derivate (Dihydro-pyrimidon-2-Derivate) mit anorganischen Säurehalogeniden, wie zum Beispiel Phosphoroxychlorid oder Phosphorpentachlorid, umsetzt, oder auch dadurch, daß man entsprechende 2-Amino-pyrimidin-Derivate mit salpetriger Säure in Gegenwart von Halogenwasserstoffsäuren umsetzt.

Die bei dem Verfahren (d) weiterhin als Ausgangsstoffe benötigten 4-Amino-thiophenole sind durch die Formel (VII) definiert. In dieser Formel haben $R^3$, $R^4$ und n vorzugsweise diejenigen Bedeutungen, die bereits im Zusammenhang mit der Beschreibung der erfindungsgemäßen Stoffe der Formel (I) vorzgusweise für diese Reste bzw. für diesen Index genannt wurden.

Die 4-Amino-thiophenole der Formel (VII) sind bekannt oder lassen sich nach im Prinzip bekannten Methoden in einfacher Weise herstellen.

Als Säurebindemittel können bei der Durchführung des Verfahrens (d) alle üblicherweise für derartige Umsetzungen verwendbaren Säureakzeptoren verwendet werden. Vorzugsweise verwendbar sind Alkali- und Erdalkalioxide, -hydroxide und -carbonate, wie Natriumhydroxid, Kaliumhydroxid, Calciumoxid, Natriumcarbonat und Kaliumcarbonat, ferner Alkali-alkoholate, -amide und -hydride, wie zum Beispiel Natrium-methylat, Natriumethylat, Kalium-tert.-butylat, Natrium-amid und Natrium-hydrid.

Als Verdünnungsmittel können bei der Durchführung des Verfahrens (d) alle üblichen inerten organischen Solventien verwendet werden. Vorzugsweise in Frage kommen Kohlenwasserstoffe, wie Benzin, Toluol und Xylol, ferner Ether, wie Dioxan, Glykoldimethylether und Diglykoldimethylether, außerdem Nitrile, wie Acetonitril, und auch stark polare Solventien, wie Dimethylsulfoxid, Sulfolan und Dimethylformamid.

Die Reaktionstemperaturen können bei der Durchführung des Verfahrens (d) innerhalb eines größeren Bereiches variiert werden. Im allgemeinen arbeitet man bei Temperaturen zwischen 0°C und 200°C, vorzugsweise zwischen 50°C und 150°C.

Die Umsetzung nach dem Verfahren (d) wird im allgemeinen unter Normaldruck vorgenommen.

Bei der Durchführung des Verfahrens (d) setzt man die Ausgangsstoffe der Formeln (VI) und (VII) im allgemeinen in angenähert äquimolaren Mengen um. Es ist jedoch auch möglich, die eine oder andere Komponente in einem größeren Überschuß zu verwenden. Die Aufarbeitung erfolgt nach üblichen Methoden.

Die bei dem Verfahren (e) als Ausgangsstoffe benötigten 2-(4-Nitro-thiophenoxy)-pyrimidin-Derivate sind durch die Formel (VIII) definiert. In dieser Formel haben $R^1$, $R^2$, $R^3$ und n vorzugsweise diejenigen Bedeutungen, die bereits im Zusammenhang mit der Beschreibung der erfindungsgemäßen

Stoffe der Formel (I) vorzugsweise für diese Reste bzw. für diesen Index genannt wurden.

Die Verbindungen der Formel (VIII) sind bekannt oder lassen sich nach im Prinzip bekannten Methoden in einfacher Weise herstellen. So erhält man Verbindungen der Formel (VIII) zum Beispiel dadurch, daß man Pyrimidin-Derivate der Formel (VI),

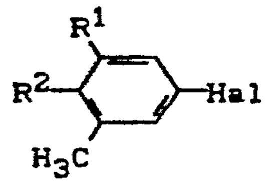

(VI)

in welcher

$R^1$, $R^2$ und Hal die oben angegebene Bedeutung haben,

mit 4-Nitro-thiophenolen der Formel (IX),

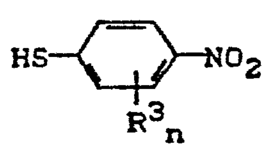

(IX)

in welcher

$R^3$ und n die oben angegebene Bedeutung haben,

in Gegenwart eines Säurebindemittels und gegebenenfalls in Gegenwart eines Verdünnungsmittels bei Temperaturen zwischen 0° C und 200° C, vorzugsweise zwischen 50° C und 150° C, umsetzt. Als Säurebindemittel und Verdünnungsmittel kommen hierbei vorzugsweise diejenigen Stoffe in Betracht,

die bereits im Zusammenhang mit dem Verfahren (d) als vorzugsweise verwendbare Säureakzeptoren und Solventien genannt wurden.

Als Reduktionsmittel kommen bei dem Verfahren (e) alle diejenigen Stoffe in Frage, die üblicherweise zur Reduktion aromatischer Nitroverbindungen eingesetzt werden. Vorzugsweise verwendbar sind elementare Metalle, wie Eisen, Zink und Zinn, ferner Metallverbindungen in niederen Wertigkeitsstufen, wie Eisen (II)- und Zinn (II)-Salze, und außerdem Nichtmetall-Verbindungen in niederen Wertigkeitsstoffen, wie z.B. Salze des Schwefelwasserstoffes, Alkalisulfite und Alkalidithionite. Im übrigen kann die Reduktion auch durch katalytische Hydrierung mit Wasserstoff in Gegenwart eines Katalysators, wie z.B. Raney-Nickel, erfolgen.

Als Verdünnungsmittel kommen bei dem Verfahren (e) alle üblichen für derartige Reduktionen geeigneten organischen Solventien in Betracht. Die Reaktionstemperaturen können innerhalb eines größeren Bereiches variiert werden. Sie entsprechen den Temperaturen, die bei analogen Reaktionen angewandt werden.

Die Durchführung der Reduktion nach dem Verfahren (e) und die Aufarbeitung des anfallenden Reaktionsgemisches erfolgen nach üblichen Methoden.

Die bei dem erfindungsgemäßen Verfahren (a, Variante α) als Reaktionskomponenten benötigten Carbonsäure-Derivate sind durch die Formel (III) eindeutig definiert. In dieser Formel haben $R^5$, $R^6$ und $R^7$ vorzugsweise diejenigen Bedeutungen, die bereits im Zusammenhang mit der Beschreibung der erfindungsgemäßen Stoffe der Formel (I) vorzugsweise für diese Reste genannt wurden. Y steht vorzugsweise für Fluor, Chlor, Brom, Benzolsulfonyl oder Toluolsulfonyl.

Die Säurehalogenide der Formel (III) sind bekannt oder lassen sich nach im Prinzip bekannten Methoden in einfacher Weise herstellen.

Als Säurebindemittel kommen bei der Umsetzung nach dem erfindungsgemäßen Verfahren (a, Variante α) alle üblichen Säureakzeptoren in Betracht. Vorzugsweise verwendbar sind tertiäre Amine, wie Triethylamin, Pyridin und N-N-Dimethyl-anilin, ferner Erdalkalimetalloxide, wie Magnesium- und Calciumoxid, außerdem Alkali- und Erdalkali-metallcarbonate, wie Natriumcarbonat, Kaliumcarbonat und Calciumcarbonat. Es ist auch möglich, die jeweiligen Anilin-Derivate der Formel (II) gleichzeitig als Säurebindemittel zu verwenden. Dazu muß die betreffende Anilin-Verbindung dann zumindest in solcher Menge eingesetzt werden, daß der freiwerdende Halogenwasserstoff gebunden werden kann.

Als Verdünnungsmittel können bei dem erfindungsgemäßen Verfahren (a, Variante $\alpha$) alle gegenüber Säurehalogeniden inerten Solventien eingesetzt werden. Vorzugsweise verwendbar sind Kohlenwasserstoffe, wie Benzin, Benzol, Toluol, Xylol und Tetralin, außerdem Ketone, wie Aceton und Methyl-isopropylketon, weiterhin Ether, wie Diethylether, Tetrahydrofuran und Dioxan, darüberhinaus Carbonsäureester, wie Ethylacetat, und auch stark polare Solventien, wie Dimethylsulfoxid und Sulfolan. Wenn die Hydrolysestabilität des Säurehalogenids es zuläßt, kann die Umsetzung auch in Gegenwart von Wasser durchgeführt werden.

Die Reaktionstemperaturen können bei der Durchführung des erfindungsgemäßen Verfahrens (a, Variante $\alpha$) innerhalb eines größeren Bereiches variiert werden. Arbeitet man ohne Lösungsmittel und Säurebindemittel, so geht man im allgemeinen so vor, daß man die Komponenten zunächst bei Temperaturen zwischen -20°C und +20°C reagieren läßt und danach auf Temperaturen zwischen 70 und 200°C erhitzt. Arbeitet man in Gegenwart eines Verdünnungsmittels und eines Säurebindemittels, so liegen die Reaktionstemperaturen im allgemeinen zwischen -20°C und +100°C, vorzugsweise zwischen 0°C und 50°C.

Das erfindungsgemäße Verfahren (a, Variante $\alpha$) wird im allgemeinen unter Normaldruck durchgeführt.

Bei der Durchführung des erfindungsgemäßen Verfahrens (a, Variante α) werden die Ausgangsstoffe der Formeln (II) und (III) im allgemeinen in angenähert äquivalenten Mengen verwendet. Es ist jedoch auch möglich, die eine oder andere Komponente in einem größeren Überschuß einzusetzen. Die Aufarbeitung erfolgt nach üblichen Methoden. Im allgemeinen geht man so vor, daß man ausgefallene Salze entfernt und das verbleibende Reaktionsgemisch durch Abziehen des Verdünnungsmittels einengt. Arbeitet man in Gegenwart von Wasser oder von mit Wasser mischbaren Solventien, so kann man auch so verfahren, daß man das Reaktionsgemisch mit Wasser verdünnt, das entstehende Gemisch absaugt oder mit einem mit Wasser wenig mischbaren organischen Lösungsmittel extrahiert, die organische Phase wäscht, einengt und den verbleibenden Rückstand gegebenenfalls üblichen Reinigungsverfahren unterwirft.

Die bei dem erfindungsgemäßen Verfahren (a, Variante β) als Ausgangsstoffe benötigten Pyrimidyl-thio-carbonsäureanilide sind durch die Formel (Ia-1) allgemein definiert. In dieser Formel haben $R^1$, $R^2$, $R^3$, $R^5$, $R^6$, $R^7$ und n vorzugsweise diejenigen Bedeutungen, die bereits im Zusammenhang mit der Beschreibung der erfindungsgemäßen Stoffe der Formel (I) vorzugsweise für diese Reste genannt wurden.

Die Verbindungen der Formel (Ia-1) sind erfindungsgemäße Stoffe und erhältlich nach Verfahren (a/Variante α).

Die zur Durchführung des erfindungsgemäßen Verfahrens (a/Variante β) weiterhin benötigten Alkylierungsmittel sind durch die Formel (IV) allgemein definiert.

In der Formel (IV) hat $R^4$ vorzugsweise diejenige Bedeutung, die bereits im Zusammenhang der Beschreibung der erfindungsgemäßen Stoffe vorzugsweise für diesen Rest genannt wurden, U steht vorzugsweise für Chlor oder Brom.

Die Alkylierungsmittel der Formel (IV) sind allgemein bekannte Verbindungen der organischen Chemie.

Als Verdünnungsmittel können bei der Durchführung des erfindungsgemäßen Verfahrens (a, Variante β) vorzugsweise diejenigen Verdünnungsmittel verwendet werden, die auch bei dem Verfahren (a, Variante α) vorzugsweise in Betracht kommen.

Die Reaktionstemperaturen können auch bei dem erfindungsgemäßen Verfahren (a, Variante β) innerhalb eines größeren Bereiches variiert werden. Im allgemeinen arbeitet man bei Temperaturen zwischen -20°C und +150°C, vorzugsweise zwischen 0 und 100°C.

Das erfindungsgemäße Verfahren (a, Variante β) wird im allgemeinen unter Normaldruck durchgeführt.

Bei der Durchführung des erfindungsgemäßen Verfahrens (a, Variante β) werden die Ausgangsstoffe der Formeln (Ia-1) und (IV) im allgemeinen in angenähert äquivalenten Mengen verwendet. Es ist jedoch auch möglich, das Alkylierungsmittel in einem größeren Überschuß einzusetzen. Die Aufarbeitung erfolgt nach üblichen Methoden.

Die zur Durchführung des erfindungsgemäßen Verfahrens (b) als Ausgangsstoffe benötigten Pyrimidyl-thio-carbonsäureanilide sind durch die Formel (Ia) allgemein definiert. In der Formel (Ia) stehen $R^1$, $R^2$, $R^3$, $R^4$, $R^5$, $R^6$, $R^7$ und n vorzugsweise für diejenigen Bedeutungen, die bereits im Zusammenhang mit der Beschreibung der erfindungsgemäßen Stoffe der Formel (I) vorzugsweise für diese Reste genannt wurden.

Die Pyrimidyl-thio-carbonsäureanilide der Formel (Ia) sind erfindungsgemäße Verbindungen und erhältlich nach Verfahren (a/Variante $\alpha$ und $\beta$).

Bei dem erfindungsgemäßen Verfahren (b) werden als Schwefelungsagentien vorzugsweise $P_4S_{10}$ und 2,4-Bis-(4-methoxyphenyl)-2,4-dithiono-1,3,2,4-dithiodiphosphetan (Lawesson-Reagenz) verwendet. Die Schwefelungsagentien sind bekannte Verbindungen.

Als Verdünnungsmittel können bei dem erfindungsgemäßen Verfahrens (b) alle für derartige Reaktionen üblichen Lösungsmittel eingesetzt werden. Vorzugsweise verwendet man Kohlenwasserstoffe wie Toluol, Xylol oder Benzol.

Die Reaktionstemperaturen können bei dem erfindungsgemäßen Verfahren (b) innerhalb eines größeren Bereiches variiert werden. Im allgemeinen arbeitet man bei Temperaturen zwischen +20°C und 200°C, vorzugsweise bei der entsprechenden Siedetemperatur des verwendeten Lösungsmittels.

Die zur Durchführung des erfindungsgemäßen Verfahrens (c) als Ausgangsstoffe benötigten Pyrimidyl-thio-carbonsäureanilide sind durch die Formel (Ib) allgemein definiert. In dieser Formel haben $R^1$, $R^2$, $R^3$, $R^4$, $R^5$, $R^6$, $R^7$ und n vorzugsweise für diejenigen Bedeutungen, die bereits im Zusammenhang mit der Beschreibung der erfindungsgemäßen Stoffe der Formel (I) vorzugsweise für diese Reste genannt wurden.

Die Pyrimidyl-thio-carbonsäureanilide der Formel (Ib) sind erfindungsgemäße Verbindungen und erhältlich nach Verfahren (b).

Die zur Durchführung des erfindungsgemäßen Verfahrens (c) weiterhin benötigten Alkylierungsmittel sind durch die Formel (V) allgemein definiert. In der Formel (V) hat $R^8$ vorzugsweise diejenige Bedeutung, die bereits im Zusammenhang mit der Beschreibung der erfindungsgemäßen Stoffe der Formel (I) vorzugsweise für diesen Rest genannt wurde, U steht vorzugsweise für Chlor oder Brom.

Die Alkylierungsmittel der Formel (V) sind bekannte Verbindungen der organischen Chemie.

Als Verdünnungsmittel und Säurebindemittel werden bei der Durchführung des erfindungsgemäßen Verfahrens (c) vorzugsweise diejenigen Verdünnungsmittel und Säurebindemittel verwendet, die auch bei dem Verfahren (a/Variante β) angewendet werden.

Die Reaktionstemperaturen können bei der Durchführung des erfindungsgemäßen Verfahrens (c) in einem größeren Bereiches variiert werden. Im allgemeinen arbeitet man bei Temperaturen zwischen -20° C und +150° C, vorzugsweise zwischen 0 und 100° C.

Bei der Durchführung des erfindungsgemäßen Verfahrens (c) werden die Ausgangsstoffe der Formeln (I) und (V) im allgemeinen in angenähert äquivalenten Mengen verwendet. Es ist jedoch auch möglich, das Alkylierungsmittel der Formel (V) in einem größeren Überschuß einzusetzen. Die Aufarbeitung erfolgt nach üblichen Methoden.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
Z.B. Ammoniumsalze und natürliche Gesteinsmehle, wie Kaoline, Tonerden, Talkum, Kreide, Quarz, Attapulgit,

Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion oder N-(2-Benzthiazolyl)-N,N'-dimethyl-harnstoff zur Unkrautbekämpfung in Getreide; 4-Amino-3-methyl-6-phenyl-1,2,4-triazin-5(4H)-on zur Unkrautbekämpfung in Zuckerrüben und 4-Amino-6-(1,1-dimethylethyl)-3-methylthio-1,2,4-triazin-5(4H)-on zur Unkrautbekämpfung in Sojabohnen, in Frage. Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro ha.

Die Herstellung und die Verwendung der erfindungsgemäßen Wirkstoffe geht aus den nachfolgenden Beispielen hervor.

Herstellungsbeispiele

Beispiel 1

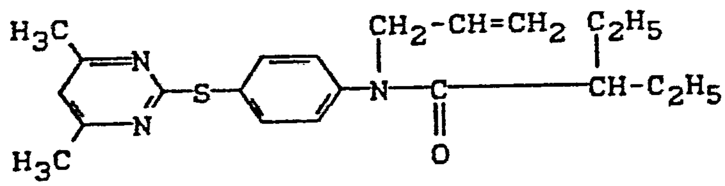

Verfahren (a-ß)

Zu 0,75 g (25 mmol) Natriumhydrid in 20 ml absolutem Dimethylformamid werden bei Raumtemperatur 6,6 g (20 mmol) 4-[4,6-Dimethyl-2-mercato)-pyrimidyl]-(2-ethyl-buttersäure)-anilid in 50 ml absolutem Dimethylformamid getropft. Nach beendeter Wasserstoffentwicklung werden 2,2 ml (25 mmol) Allylbromid zugefügt und die Reaktionsmischung bei Raumtemperatur unter Kontrolle durch Dünnschichtchromatographie gerührt. Nach dem Zutropfen von wenig Eiswasser wird die Reaktionsmischung in Wasser gegossen. Anschließend wird das Reaktionsprodukt mit Methylenchlorid extrahiert, mit Natriumsulfat getrocknet und das Lösungsmittel im Vakuum abdestilliet.

Man erhält nach Säulenchromatographie mit Cyclohexan/-Essigester (2:1) 3,62 g (49 % der Theorie) 4-[4,6-Dimethyl-2-mercapto)-pyrimidyl]-N-allyl-(2-ethylbuttersäure)-anilid vom Schmelzpunkt 97-98° C.

Beispiel 2

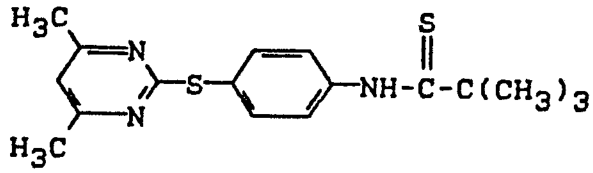

Verfahren (b)

9,5 g (30 mmol) 4-[(4,6-Dimethyl-2-mercapto-pyrimidyl]-pivaloylanilid und 6,6 g (16,5 mmol) Lawesson-Reagenz werden in 30 ml absolutem Toluol 3 Stunden unter Rückfluß erhitzt. Nach dem Abkühlen auf Raumtemperatur wird die Reaktionsmischung mit Cyclohexan/Essigester (2:1) chromatographiert.

Man erhält 4,55 g (46 % der Theorie) 4-[(4,6-Dimethyl-2-mercapto)-pyrimidyl]-pivaloylthioanilid vom Schmelzpunkt 165-167° C.

Beispiel 3

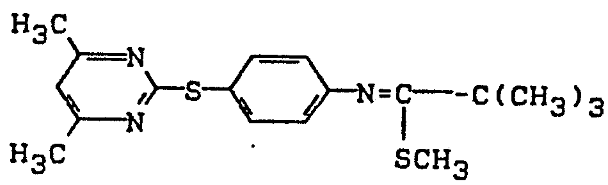

Verfahren (c)

Zu 0,9 g (30 mmol) Natriumhydrid in 20 ml absolutem Dimethylformamid werden unter Stickstoffatmosphäre 6,62 g (20 mmol) 4-[(4,6-Dimethyl-2-mercapto-pyrimidyl]-pivalo-

ylthioanilid in 30 ml absolutem Dimethylformamid getropft. Nach beendeter Wasserstoffentwicklung wird unter Eiskühlung 2,5 ml (40 mmol) Methyljodid zugefügt. Der Reaktionsverlauf wird unter Kontrolle durch Dünnschichtchromatographie verfolgt. Nach vorsichtiger Hydrolyse des Natriumhydridüberschusses wird die Reaktionsmischung in Wasser gegossen. Anschließend wird das Reaktionsprodukt mit Methylenchlorid extrahiert, mit Natriumsulfat getrocknet, und das Lösungsmittel wird im Vakuum abdestilliert.

Man erhält 4,2 g (60,8 % der Theorie) 4-[(4,6-Dimethyl-2-mercapto)-pyrimidyl]-methylthio-tert.-butyliminoanilid vom Schmelzpunkt 136-137° C (Umkristallisation aus Essigester/n-Hexan).

In entsprechender Weise und gemäß den allgemeinen Angaben zur Herstellung erhält man die in der folgenden Tabellen aufgeführten Pyrimidyl-thio-carbonsäure-anilide der Formel (I):

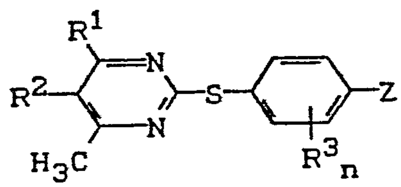

(I)

Tabelle 3

Z = 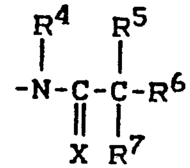

| Bsp. Nr. | $R^1$ | $R^2$ | $R^3$ | $R^4$ | $R^5$ | $R^6$ | $R^7$ | X | Schmelzpunkt °C |
|---|---|---|---|---|---|---|---|---|---|
| 4 | $CH_3$ | H | H | $CH_3$ | $CH_3$ | $CH_3$ | $CH_3$ | O | 127-128 |
| 5 | $CH_3$ | H | H | $-(CH_2)_2-$ | | H | H | O | 143-144 |
| 6 | $CH_3$ | H | H | $-(CH_2)_2-$ | | $CH_3$ | $CH_3$ | O | 143 |
| 7 | $CH_3$ | H | H | $-(CH_2)_3-$ | | H | H | O | 134-135 |
| 8 | $CH_3$ | H | H | $-CH_2-CH=CH_2$ | $CH_3$ | $CH_3$ | H | O | 97- 98 |
| 9 | $CH_3$ | H | H | H | $CH_3$ | $CH_3$ | $C_2H_5$ | S | 117-118 |
| 10 | $CH_3$ | H | H | H | $CH_3$ | $CH_3$ | $n-C_3H_7$ | S | 109-111 |
| 11 | $CH_3$ | H | H | H | $CH_3$ | $CH_3$ | $CH=CH_2$ | S | 153-154 |
| 12 | $CH_3$ | H | H | H | $CH_3$ | $-CCl_2-CH_2-$ | | S | 120-121 |
| 13 | $CH_3$ | H | H | H | $CH_3$ | $-(CH_2)_4-$ | | S | 112-113 |
| 14 | $CH_3$ | H | H | H | $CH_3$ | $CH_3$ | H | S | 183-184 |
| 15 | $CH_3$ | H | H | H | $C_2H_5$ | $C_2H_5$ | H | S | 155-156 |
| 16 | $CH_3$ | H | H | H | $CH_3$ | $CH_3$ | $-CH=CH_2$ | S | 153-154 |
| 17 | $CH_3$ | H | H | $-(CH_2)_2-$ | | H | H | S | 121-123 |
| 18 | $CH_3$ | H | H | $-(CH_2)_2-$ | | $CH_3$ | $CH_3$ | S | |

Tabelle 3 (Fortsetzung)

| Bsp. Nr. | $R^1$ | $R^2$ | $R^3$ | $R^4$ | $R^5$ | $R^6$ | $R^7$ | X | Schmelzpunkt °C |
|---|---|---|---|---|---|---|---|---|---|
| 19 | $CH_3$ | H | H | $-(CH_2)_3-$ | | H | H | S | 169-170 |
| 20 | $CH_3$ | H | -3Cl | H | $CH_3$ | $CH_3$ | $CH_3$ | S | |
| 21 | $CH_3$ | H | $-3CH_3$ | H | $CH_3$ | $CH_3$ | $CH_3$ | S | 105 |
| 22 | $CH_3$ | H | $-3OCH_3$ | H | $CH_3$ | $CH_3$ | $CH_3$ | S | |
| 23 | $CH_3$ | $CH_3$ | H | H | $CH_3$ | $CH_3$ | $CH_3$ | S | |
| 24 | $CH_3$ | $C_2H_5$ | H | H | $CH_3$ | $CH_3$ | $CH_3$ | S | |
| 25 | H | H | H | H | $CH_3$ | $CH_3$ | $CH_3$ | S | 102 |
| 26 | $CH_3$ | H | H | H | $CH_3$ | $CH_3$ | $i-C_3H_7$ | S | 121 |
| 27 | $CH_3$ | H | $3-OCH_3$ | $-(CH_2)_2-$ | | $CH_3$ | $CH_3$ | O | 127 |
| 28 | H | $CH_3$ | H | $-(CH_2)_2-$ | | $CH_3$ | $CH_3$ | O | 121 |
| 29 | H | H | $3-CH_3$ | $-(CH_2)_2-$ | | $CH_3$ | $CH_3$ | O | 106 |
| 30 | H | H | H | H | $CH_3$ | $CH_3$ | $C_2H_5$ | S | 76 |
| 31 | H | H | H | H | $CH_3$ | $CH_3$ | $i-C_3H_7$ | S | 100 |
| 32 | H | $CH_3$ | H | H | $CH_3$ | $CH_3$ | $CH_3$ | S | 120 |
| 33 | H | $CH_3$ | H | H | $CH_3$ | $CH_3$ | $C_2H_5$ | S | 70 |
| 34 | H | $CH_3$ | H | H | $CH_3$ | $CH_3$ | $i-C_3H_7$ | S | 88 |

Tabelle 4

Z = 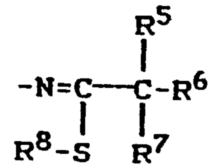

| Bsp. Nr. | $R^1$ | $R^2$ | $R^3$ | $R^5$ | $R^6$ | $R^7$ | $R^8$ | Schmelzpunkt °C |
|---|---|---|---|---|---|---|---|---|
| 35 | $CH_3$ | H | H | $CH_3$ | $CH_3$ | $CH_3$ | $C_2H_5$ | 123-124 |
| 36 | $CH_3$ | H | H | $CH_3$ | $CH_3$ | $CH_3$ | $i-C_3H_7$ | 125-126 |
| 37 | $CH_3$ | H | H | $CH_3$ | $CH_3$ | $CH_3$ | $CH_2-CH=CH_2$ | 58- 59 |
| 38 | $CH_3$ | H | H | $CH_3$ | $CH_3$ | $CH_3$ | $CH_2-C\equiv CH$ | 94- 95 |
| 39 | $CH_3$ | H | 3-Cl | $CH_3$ | $CH_3$ | $CH_3$ | $CH_3$ | |
| 40 | $CH_3$ | H | $3-CH_3$ | $CH_3$ | $CH_3$ | $CH_3$ | $CH_3$ | |
| 41 | $CH_3$ | H | $3-OCH_3$ | $CH_3$ | $CH_3$ | $CH_3$ | $CH_3$ | |
| 42 | $CH_3$ | $CH_3$ | H | $CH_3$ | $CH_3$ | $CH_3$ | $CH_3$ | |
| 43 | $CH_3$ | $C_2H_5$ | H | $CH_3$ | $CH_3$ | $CH_3$ | $CH_3$ | |
| 44 | H | H | H | $CH_3$ | $CH_3$ | $CH_3$ | $CH_3$ | |
| 45 | H | Cl | H | $CH_3$ | $CH_3$ | $CH_3$ | $CH_3$ | 105 |
| 46 | $CH_3$ | H | H | $CH_3$ | $CH_3$ | $i-C_3H_7$ | $CH_3$ | 65 |

Tabelle 4 (Fortsetzung)

Z = 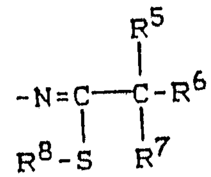

| Bsp. Nr. | $R^1$ | $R^2$ | $R^3$ | $R^5$ | $R^6$ | $R^7$ | $R^8$ | Schmelzpunkt °C |
|---|---|---|---|---|---|---|---|---|
| 47 | H | $CH_3$ | H | $CH_3$ | $CH_3$ | $CH_3$ | $CH_3$ | 100 |
| 48 | H | $CH_3$ | H | $CH_3$ | $CH_3$ | $CH_3$ | $C_2H_5$ | 53 |
| 49 | H | $CH_3$ | H | $CH_3$ | $CH_3$ | $CH_3$ | $-CH_2-CH=CH_2$ | 42 |
| 50 | H | $CH_3$ | $3-CH_3$ | $CH_3$ | $CH_3$ | $CH_3$ | $CH_3$ | 76 |
| 51 | H | $CH_3$ | $3-CH_3$ | $CH_3$ | $CH_3$ | $CH_3$ | $-CH_2-CH=CH_2$ | Öl |
| 52 | H | H | H | $CH_3$ | $CH_3$ | $CH_3$ | $-CH_2-CH=CH_2$ | 50 |
| 53 | H | H | H | $CH_3$ | $CH_3$ | $CH_3$ | $-CH_2-C\equiv CH$ | 116 |
| 54 | H | H | H | $CH_3$ | $CH_3$ | $C_2H_5$ | $-CH_2-CH=CH_2$ | 44 |
| 55 | H | H | H | $CH_3$ | $CH_3$ | $i-C_3H_7$ | $-CH_2-CH=CH_2$ | 60 |

**Beispiel A**

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.
Es bedeuten:

0 % = keine Wirkung (wie unbehandelte Kontrolle)
100 % = totale Vernichtung

In diesem Test zeigen beispielsweise die Verbindungen gemäß den Herstellungsbeispielen 2, 6, 9, 10, 13 und 33 eine sehr gute herbizide Wirkung gegen Unkräuter wie z.B. Chenopodium, Helianthus, Portulak und Sinapis bei sehr guter Nutzpflanzenverträglichkeit, insbesondere bei Gerste.

## Patentansprüche

1. Pyrimidyl-thio-carbonsäureanilide Formel (I),

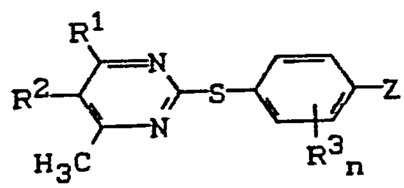

in welcher

$R^1$ für Wasserstoff, Halogen, für Alkyl, Alkoxy oder Alkylthio mit jeweils 1 bis 6 Kohlenstoffatomen, Trifluormethyl, oder für gegebenenfalls einfach oder mehrfach, gleich oder verschieden durch Halogen, Methyl, Ethyl, Methoxy oder Trifluormethyl substituiertes Phenyl steht,

$R^2$ für Wasserstoff, Halogen, Alkyl mit 1 bis 6 Kohlenstoffatomen oder Trifluormethyl steht,

$R^3$ für Halogen, Methyl oder Methoxy steht,

n für eine ganze Zahl 0, 1 oder 2 steht, und

Z für den Rest

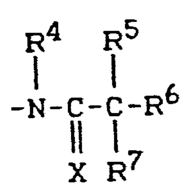 oder 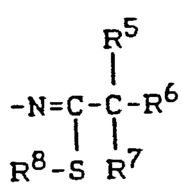

steht,

wobei

X für Sauerstoff oder Schwefel steht,

$R^4$ für Wasserstoff, Hydroxyl, für Alkyl oder Alkoxy mit jeweils 1 bis 6 Kohlenstoffatomen, für Alkenyl oder Alkinyl mit jeweils 3 bis 6 Kohlenstoffatomen steht, mit der Maßgabe, daß $R^4$ nicht für Wasserstoff steht, wenn X für Sauerstoff steht,

$R^5$ für Wasserstoff, Halogen oder für gegebenenfalls einfach oder mehrfach durch Halogen substituiertes Alkyl mit 1 bis 6 Kohlenstoffatomen steht oder

$R^4$ und $R^5$ gemeinsam mit dem Kohlenstoff und dem Stickstoff, an die sie gebunden sind, einen 3- bis 8-gliedrigen heterocyclischen Ring bilden können, der gegebenenfalls einfach oder mehrfach, gleich oder verschieden durch Halogen, Methyl oder Ethyl substituiert ist und welcher eine oder mehrere Doppelbindungen enthalten kann,

$R^6$ und $R^7$ unabhängig voneinander für Halogen oder für gegebenenfalls einfach oder mehrfach durch Halogen substituiertes Alkyl mit 1 bis 6 Kohlenstoffatomen stehen oder

$R^6$ und $R^7$ gemeinsam mit dem Kohlenstoffatom, an das sie gebunden sind, einen 3- bis 8-gliedrigen Ring bilden, der außer Kohlenstoff auch Sauerstoff- und/oder Schwefelatome als Ringglieder enthalten kann und der gegebenenfalls einfach oder mehrfach, gleich oder verschieden durch Halogen, Methyl oder Ethyl substituiert ist und welcher eine oder mehrere Doppelbindungen enthalten kann, und

$R^8$ für Wasserstoff, für gegebenenfalls einfach oder mehrfach durch Halogen substituiertes Alkyl mit 1 bis 6 Kohlenstoffatomen, für Alkenyl oder Alkinyl mit jeweils 3 bis 6 Kohlenstoffatomen steht.

2. Pyrimidyl-thio-carbonsäureanilide der Formel (I) gemäß Anspruch 1, bei welchen

$R^1$ für Wasserstoff, Fluor, Chlor, Brom, für jeweils geradkettiges oder verzweigtes Alkyl, Alkoxy oder Alkylthio mit jeweils 1 bis 4 Kohlenstoffatomen oder für Trifluormethyl steht,

$R^2$ für Wasserstoff, Fluor, Chlor, Brom, geradkettiges oder verzweigtes Alkyl mit 1 bis 4 Kohlenstoffatomen oder Trifluormethyl steht,

$R^3$ für Fluor, Chlor, Brom, Methyl oder Methoxy steht,

n für eine ganze Zahl 0, 1 oder 2 steht und

Z für den Rest

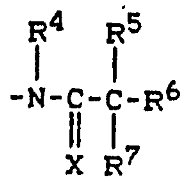 oder 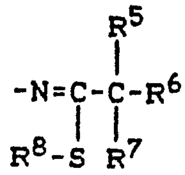

steht,

wobei

X für Sauerstoff oder Schwefel steht,

$R^4$ für Wasserstoff, Hydroxyl, für jeweils geradkettiges oder verzweigtes Alkyl oder Alkoxy mit jeweils 1 bis 4 Kohlenstoffatomen, für jeweils geradkettiges oder verzweigtes Alkenyl oder Alkinyl mit jeweils 3 bis 4 Kohlenstoffatomen steht,

mit der Maßgabe, daß $R^4$ nicht für Wasserstoff steht, wenn X für Sauerstoff steht,

$R^5$ für Wasserstoff, Fluor, Chlor, Brom oder für geradkettiges oder verzweigtes gegebenenfalls einfach bis neunfach, gleich oder verschieden durch Fluor, Chlor und Brom substituiertes Alkyl mit 1 bis 4 Kohlenstoffatomen steht oder

$R^4$ und $R^5$ gemeinsam mit dem Kohlenstoff und dem Stickstoff, an die sie gebunden sind, einen 3- bis 8-gliedrigen heterocyclischen Ring bilden, der gegebenenfalls einfach bis zwölffach, gleich oder verschieden durch Methyl und Ethyl substituiert ist und welcher eine, zwei, drei oder vier Doppelbindungen enthalten kann,

$R^6$ und $R^7$ unabhängig voneinander für Fluor, Chlor, Brom oder für geradkettiges oder verzweigtes gegebenenfalls einfach bis neunfach, gleich oder verschieden durch Fluor und Chlor substituiertes Alkyl mit 1 bis 4 Kohlenstoffatomen stehen oder

$R^6$ und $R^7$ gemeinsam mit dem Kohlenstoffatom, an das sie gebunden sind, einen 3- bis 8-gliedrigen Ring bilden, der außer Kohlenstoff gegebenenfalls 1 oder 2 Sauerstoff- und/oder Schwefelatome als Ringglieder enthält und der gegebenenfalls einfach bis zwölffach, gleich oder verschieden durch Fluor, Chlor, Methyl oder Ethyl substituiert ist und welcher eine oder zwei Doppelbindungen enthalten kann, und

$R^8$ für Wasserstoff, für gegebenenfalls einfach bis fünffach, gleich oder verschieden durch Fluor oder Chlor substituiertes geradkettiges oder verzweigtes Alkyl mit 1 bis 4 Kohlenstoffatomen, oder für jeweils geradkettiges oder verzweigtes Alkenyl oder Alkinyl mit jeweils 3 bis 6 Kohlenstoffatomen steht.

3. Pyrimidyl-thio-carbonsäureanilide der Formel (I) gemäß Anspruch 1, in welcher

$R^1$ für Wasserstoff, Fluor, Chlor, Brom, Methyl, Ethyl, Methoxy, Ethoxy, Methylthio, Ethylthio oder Trifluormethyl steht,

$R^2$ für Wasserstoff, Fluor, Chlor, Brom, Methyl, Ethyl oder Trifluormethyl steht,

$R^3$ für Fluor, Chlor, Brom, Methyl oder Methoxy steht,

n für eine ganze Zahl 0, 1 oder 2 steht,

Z für den Rest

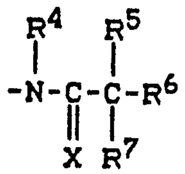 oder 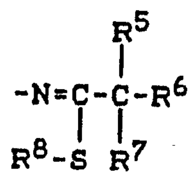

steht,

wobei

X für Sauerstoff oder Schwefel steht,

$R^4$ für Wasserstoff, Hydroxyl, Methyl, Ethyl, Methoxy, Ethoxy, Allyl oder Propargyl steht,

mit der Maßgabe, daß $R^4$ nicht für Wasserstoff steht, wenn X für Sauerstoff steht,

$R^5$ für Wasserstoff, Fluor, Chlor, Brom, Methyl oder Ethyl steht, oder

$R^4$ und $R^5$ gemeinsam mit dem Kohlenstoff bzw. dem Stickstoff, an die sie gebunden sind, einen 3- bis 8-gliedrigen heterocyclischen Ring bilden, der gegebenenfalls einfach bis sechsfach, gleich oder verschieden durch Methyl oder Ethyl substituiert ist,

$R^6$ und $R^7$ unabhängig voneinander für Fluor, Chlor, Brom, Methyl, Ethyl oder iso-Propyl stehen, oder

$R^6$ und $R^7$ gemeinsam mit dem Kohlenstoffatom, an das sie gebunden sind, einen 3- bis 8-gliedrigen Ring bilden, der außer Kohlenstoff gegebenenfalls 1 oder 2 Sauerstoff und/oder Schwefelatome als Ringglieder enthält und der gegebenenfalls einfach bis dreifach, gleich oder verschieden durch Fluor, Chlor, Methyl oder Ethyl substituiert ist, und

$R^8$ für Wasserstoff, für gegebenenfalls einfach bis fünffach, gleich oder verschieden durch Fluor oder Chlor substituiertes Methyl, Ethyl oder iso-Propyl, für Allyl oder Propargyl steht.

4. Verfahren zur Herstellung von Pyrimidyl-thio-carbonsäureaniliden der Formel (I),

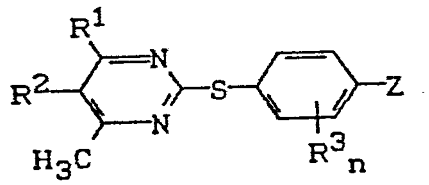

in welcher

$R^1$ für Wasserstoff, Halogen, für Alkyl, Alkoxy oder Alkylthio mit jeweils 1 bis 6 Kohlenstoffatomen, Trifluormethyl, oder für gegebenenfalls einfach oder mehrfach, gleich oder verschieden durch Halogen, Methyl, Ethyl, Methoxy oder Trifluormethyl substituiertes Phenyl steht,

$R^2$ für Wasserstoff, Halogen, Alkyl mit 1 bis 6 Kohlenstoffatomen oder Trifluormethyl steht,

$R^3$ für Halogen, Methyl oder Methoxy steht,

n für eine ganze Zahl 0, 1 oder 2 steht, und

Z für den Rest

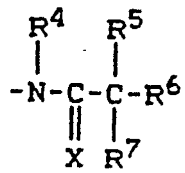 oder 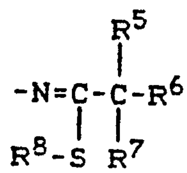

steht,

wobei

X für Sauerstoff oder Schwefel steht,

$R^4$ für Wasserstoff, Hydroxyl, für Alkyl oder Alkoxy mit jeweils 1 bis 6 Kohlenstoffatomen, für Alkenyl oder Alkinyl mit jeweils 3 bis 6 Kohlenstoffatomen steht, mit der Maßgabe, daß $R^4$ nicht für Wasserstoff steht, wenn X für Sauerstoff steht,

$R^5$ für Wasserstoff, Halogen oder für gegebenenfalls einfach oder mehrfach durch Halogen substituiertes Alkyl mit 1 bis 6 Kohlenstoffatomen steht oder

$R^4$ und $R^5$ gemeinsam mit dem Kohlenstoff und dem Stickstoff, an die sie gebunden sind, einen 3-

bis 8-gliedrigen heterocyclischen Ring bilden können, der gegebenenfalls einfach oder mehrfach, gleich oder verschieden durch Halogen, Methyl oder Ethyl substituiert ist und welcher eine oder mehrere Doppelbindungen enthalten kann,

$R^6$ und $R^7$ unabhängig voneinander für Halogen oder für gegebenenfalls einfach oder mehrfach durch Halogen substituiertes Alkyl mit 1 bis 6 Kohlenstoffatomen stehen oder

$R^6$ und $R^7$ gemeinsam mit dem Kohlenstoffatom, an das sie gebunden sind, einen 3- bis 8-gliedrigen Ring bilden, der außer Kohlenstoff auch Sauerstoff- und/oder Schwefelatome als Ringglieder enthalten kann und der gegebenenfalls einfach oder mehrfach, gleich oder verschieden durch Halogen, Methyl oder Ethyl substituiert ist und welcher eine oder mehrere Doppelbindungen enthalten kann, und

$R^8$ für Wasserstoff, für gegebenenfalls durch Halogen substituiertes geradkettiges oder verzweigtes Alkyl mit 1 bis 6 Kohlenstoffatomen, für jeweils geradkettiges oder verzweigtes Alkenyl oder Alkinyl mit jeweils 3 bis 6 Kohlenstoffatomen steht,

dadurch gekennzeichnet, daß man

(a) Pyrimidyl-thio-carbonsäureanilide der Formel (Ia),

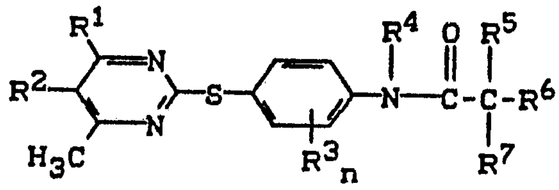

(Ia)

in welcher

$R^1$, $R^2$, $R^3$, $R^4$, $R^5$, $R^6$, $R^7$ und n die oben angegebene Bedeutung haben,

erhält, wenn man

(a-α) wenn man 4-(Pyrimidyl-thio)-aniline der Formel (II),

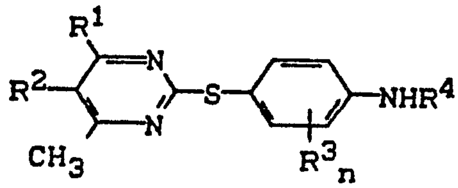

(II)

in welcher

$R^1$, $R^2$, $R^3$, $R^4$ und n die oben angegebene Bedeutung haben,

mit Carbonsäure-Derivaten der Formel (III),

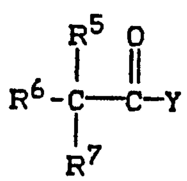

(III)

in welcher

$R^5$, $R^6$ und $R^7$ die oben angegebene Bedeutung haben und

Y für Hydroxyl, Halogen, Acyloxy, Benzolsulfonyl oder Toluolsulfonyl steht,

gegebenenfalls in Gegenwart eines Verdünnungsmittels sowie gegebenenfalls in Gegenwart eines Säurebindemittels umsetzt,

oder

(a-β) die nach Verfahren (a-α) erhältlichen Pyrimidyl-thio-carbonsäureanilide der Formel (Ia-1),

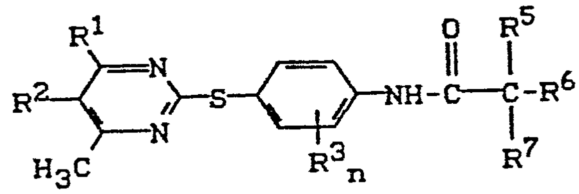

(Ia-1)

in welcher

$R^1$, $R^2$, $R^3$, $R^5$, $R^6$, $R^7$ und n die oben angegebene Bedeutung haben,

mit Alkylierungsmitteln der Formel (IV)

$$R^4 - U \qquad (IV)$$

in welcher

$R^4$ die oben angegebene Bedeutung hat und

U für eine elektronenanziehende Abgangsgruppe steht,

gegebenenfalls in Gegenwart eines Verdünnungsmittels sowie gegebenenfalls in Gegenwart eines Säurebindemittels umsetzt,

oder daß man

(b) Pyrimidyl-thio-carbonsäureanilide der Formel (Ib),

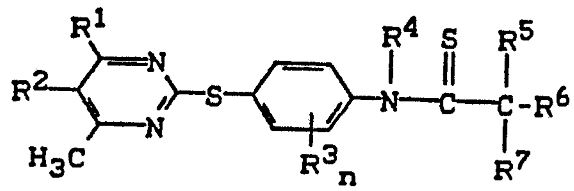

(Ib)

in welcher

$R^1$, $R^2$, $R^3$, $R^4$, $R^5$, $R^6$, $R^7$ und n die oben angegebene Bedeutung haben,

erhält, wenn man die nach Verfahren (a/Variante α und β) erhältlichen Pyrimidyl-thio-carbonsäureanilide der Formel (Ia),

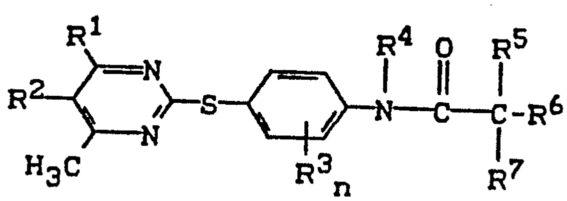

(Ia)

in welcher

$R^1$, $R^2$, $R^3$, $R^4$, $R^5$, $R^6$, $R^7$ und n die oben angegebene Bedeutung haben,

mit Schwefelungsagentien in Gegenwart eines Verdünnungsmittels umsetzt,

oder daß man

(c) Pyrimidyl-thio-carbonsäureanilide der Formel (Ic),

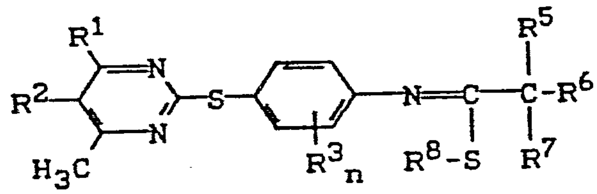

(Ic)

in welcher

$R^1$, $R^2$, $R^3$, $R^5$, $R^6$, $R^7$, $R^8$ und n die oben angegebene Bedeutung haben,

erhält, wenn man die nach Verfahren (b) erhältlichen Pyrimidyl-thio-carbonsäureanilide der Formel (Ib), mit Alkylierungsmitteln der Formel (V),

$$R^8 - U \qquad (V)$$

in welcher

$R^8$ und U die oben angegebene Bedeutung haben,

gegebenenfalls in Gegenwart eines Verdünnungsmittels und gegebenenfalls in Gegenwart eines Säurebindemittels umsetzt.

5. Herbizide Mittel, gekennzeichnet durch einen Gehalt an mindestens einem Pyrimidyl-thio-carbonsäureanilid der Formel (I) gemäß den Ansprüchen 1 bis 4.

6. Verfahren zur Bekämpfung von Unkräutern, dadurch gekennzeichnet, daß man Pyrimidyl-thio-carbonsäureanilide der Formel (I) gemäß den Ansprüchen 1 bis 4 auf die Unkräuter und/oder ihren Lebensraum einwirken läßt.

7. Verwendung von Pyrimidyl-thio-carbonsäureaniliden der Formel (I) gemäß den Ansprüchen 1 bis 4 zur Bekämpfung von Unkräutern.

8. Verfahren zur Herstellung von herbiziden Mitteln, dadruch gekennzeichnet, daß man Pyrimidyl-thio-carbonsäureanilide der Formel (I) gemäß den Ansprüche 1 bis 4 mit Streckmitteln und/oder oberflächenaktiven Substanzen vermischt.

Europäisches Patentamt

# EUROPÄISCHER RECHERCHENBERICHT

0229283

Nummer der Anmeldung

EP 86116659.3

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl 4) |
|---|---|---|---|
| P,X | EP - A2 - 0 168 608 (BAYER)<br>* Ansprüche 1-7 *<br>-- | 1-8 | C 07 D 239/38<br>C 07 D 401/12<br>C 07 D 403/12<br>A 01 N 43/54 |
| A | DD - A - 109 170 (LANG et al.)<br>* Ansprüche 1-3; Tabelle 1, laufende Nr. 55 *<br>---- | 1,5-8 | |

| RECHERCHIERTE SACHGEBIETE (Int Cl 4) |
|---|
| C 07 D 239/00<br>C 07 D 401/00<br>C 07 D 403/00 |

Der vorliegende Recherchenbericht wurde für alle Patentansprüche erstellt

| Recherchenort | Abschlußdatum der Recherche | Prüfer |
|---|---|---|
| WIEN | 20-03-1987 | LUX |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82